# EUROPEAN PATENT APPLICATION

(11) **EP 4 023 156 A1**
(43) Date of publication of application: **06.07.2022**
(21) Application number: 20217994.1
(22) Date of filing: 31.12.2020
(51) Int. Cl.: A61B 5/11, A61B 5/0537, A61B 5/00

(54) **A SLEEP THERAPY SYSTEM**

(71) Applicant: Koninklijke Philips N.V., 5656 AG Eindhoven (NL)
(72) Inventor: KAHLERT, Joachim Johannes, 5656 AE Eindhoven (NL); DOS SANTOS DA FONSECA, Pedro Miguel Ferreira, 5656 AE Eindhoven (NL); GRASSI, Angela, 5656 AE Eindhoven (NL); WILLARD, Nicolaas Petrus, 5656 AE Eindhoven (NL)
(74) Representative: Philips Intellectual Property & Standards

(57) **Abstract**

A sleep therapy system comprises a sensor arrangement for detecting physiological parameters of a subject, a detection system for detecting a sleeping position of the subject and an alert system for instructing a change in sleep position of the subject. From the monitored physiological parameters, fluid accumulation is identified in a region of the body of the subject. The alert system is controlled to instruct a change in sleep position thereby to reverse the fluid accumulation.

## Description

### FIELD OF THE INVENTION

This invention relates to sleep therapy systems, for treating sleep apnea, and including at least a positional therapy function.

### BACKGROUND OF THE INVENTION

Sleep apnea is a multifactorial disorder. Age, gender, body weight, cardiac insufficiencies, pulmonary diseases, seasonal infections, and life style contribute to, and worsen, the disorder.

Obstructive sleep apnea (OSA) is characterized by a narrowing and collapse of the upper airway. Anatomical properties and features contribute mainly to the collapse. Beside static anatomical features, also dynamic alterations of the upper airway impede the narrowing of the airway. In particular, the gravitational displacement and swelling of soft tissue in the airway (palate, tongue, and epiglottis) narrow the airway and increase the Apnea Hypopnea index (AHI).

Central sleep apnea (CSA) is characterized by a decrease of the respiration drive. The decrease of neural activity leads to a decline in tidal volume, a full cessation of airflow or a periodic breathing (Cheyne-Stokes Breathing).

The first line therapy in OSA is a positive airway pressure therapy (PAP) and the first line therapy in CSA is an adaptive support ventilation by an Average Volume Assure Pressure Support (AVAPS) device. These therapies are effective to rebalance the airflow. However, the continuous positive pressure in these OSA and CSA therapies can cause a further physiological stress.

An alternative, less invasive, treatment approach for sleep apnea is to control the posture of the patient during sleep, in particular to ensure they sleep on their side rather than on their back.

A traditional method of positional sleep training is known as the "Tennis Ball Technique". With this technique, a tennis ball is sewn into the back of a shirt worn to bed by the subject so that the subject finds sleeping on his or her back uncomfortable.

Electronic positional sleep therapy devices are also well known for treating positional sleep apnea. Typical positional sleep therapy device use an accelerometer system to detect whether a subject is sleeping on his or her back and provide vibratory feedback to the subject that prompts the subject to change his or her position.

The known sleep therapy systems are for providing position control based on detection of sleep apnea events. However, they do not provide therapy which addresses other physiological problems, in particular problems relating to fluid accumulation and fluid shift.

Fluid accumulation and fluid shift are known physiological alterations in sleep. The body compartments being effected are the upper airway (nasal, pharyngeal airway), pulmonary airway (bronchi, alveoli) and the heart chambers and vascular structures in the thorax. The fluid uptake is one of the causes of sleep disordered breathing SDB.

The primary cause is the body position in sleep. When going to bed, the transition from an upright body position to lying down changes the hydrostatic pressure and gravitational forces. In persons with underlying health conditions like sleep apnea, but also heart failure (for instance CHF) and pulmonary disease (for instance COPD), the fluid accumulation is more severe and needs a preventing therapy. The affected anatomical structures and the amount of fluid shift depend on the type of comorbidity and its severity.

The fluid shift in sleep when a person is lying down can be classified into three Cause-Symptom-Relationships:
(i) Rostral fluid shift: the change of the hydrostatic pressure difference between upper airway and lower body compartments (legs) is the driving force to shift liquid into the upper airway causing swelling. The amount of volume accumulation in different parts of the body depends on the height position of the heart relative to the whole body. In a left lateral body position the heart is in a low position and the hydrostatic pressure is higher than in the right lateral position. This relative height difference, and hence the difference in hydrostatic pressure, causes a higher venous return flow. This is the major cause of the volume overloading, for example in coronary heart failure (CHF) patients. Such volume overloading causes stretching of the heart chamber. The baroreceptor and Bainbridge receptors in the central veins and in the right atrium trigger a sympathetic response causing a hemodynamic alteration (which is manifested as a change in heart rate and stroke volume). A positional therapy itself may thus influence the rostral fluid shift.
(ii) Negative inspiratory pressure in an apnea and hypopnea event: The negative pressure in the lung increases the transmural pressure in the alveoli and increases the venous return flow into the right atrium. This higher pressure is the cause of liquid accumulation in the lung.
(iii) Hyperinflation in COPD patients. Due to the high collapsibility of the tiny bronchi and alveoli, a patient for example compensates by a positive-end-expiratory-pressure (PEEP). This high PEEP is the major cause for pulmonary hypertension and lung edema. The high PEEP hampers the venous return flow into the atrium. This is a cause for a liquid accumulation in the lower extremities during the day and the rostral fluid shift during sleep. The positive end expiratory pressure (PEEP) in the lung impedes the pulmonary circulation. PEEP increases the pulmonary vascular resistance which is the cause of the volume overloading in the heart.

A secondary cause for an increased fluid shift is the side effect of a sleep apnea therapy. The positive pressure by a positive applied pressure (PAP) device to the airway increases the transmural pressure in the upper and pulmonary airway. The transmural pressure changes are a driving force for a liquid uptake in the lung and a volume accumulation the heart chambers and blood vessels. The extrinsic positive pressure in the lung has a similar physiological effect as the PEEP in COPD patients.

The intrathoracic pressure also modulates the systemic circulation; the venous return (preload at right atrium) will change and the stroke volume of the left ventricle will change. The intrathoracic pressure thus has implications on the cardiac preload and afterload. The cardiac system responds by a change in heart rate and stroke volume, in particular an increased heart rate and an increased systemic arterial pressure. Furthermore, the pressure is the cause of an increased vascular resistance, which leads, in particular in heart failure patients, to a cardiac volume overload.

In patients who suffer from a singular sleep apnea disorder, the therapy target is to eliminate the stress caused by the apnea event. The secondary cause explained above, namely the therapy device related stress, can generally be neglected in this patient cohort.

However, in OSA patients with cardiac (CHF) and pulmonary comorbidities (COPD, edema), the physiological stress caused by e.g. a CPAP therapy itself is a medical concern and should ideally be considered as well. This leads to different therapy choices (such as different settings for the therapy system) compared with patients not suffering from a comorbid health condition.

Thus, it would be desirable for a positional sleep therapy system to adapt in response to fluid accumulation and fluid shifts, for example which may be caused by a sleep apnea therapy which is being used in combination with the positional sleep therapy system.

### SUMMARY OF THE INVENTION

The invention is defined by the claims.

According to examples in accordance with an aspect of the invention, there is provided a sleep therapy system, comprising:
a sensor arrangement for detecting physiological parameters of a subject;
a detection system for detecting a sleeping position of the subject;
an alert system for instructing a change in sleep position of the subject; and
a controller,
wherein the controller is adapted to:
   identify, from the physiological parameters, fluid accumulation in a region of the body of the subject; and
   control the alert system to instruct a change in sleep position thereby to remedy the fluid accumulation.

This sleep therapy system uses position therapy to address fluid accumulation in a region of the body (such as the lungs, heart or airway). Physiologically, any change of a transmural pressure in the vascular structures contributes to a fluid shift. This can for example arise when performing other sleep therapy approaches, such as positive airway pressure therapies in conjunction with the position therapy. The position therapy may thus be one part of an overall therapy solution including other types of therapy, such as a PAP therapy (such as CPAP) or AVAPS therapy.

The type of fluid accumulation (shift, tissue swelling or volume overloading) which will be experienced by a patient depends on the type(s) of comorbidity and the type of fluid shift is specific for the comorbidity. The choice of therapy and the therapy solution can thus be based on the known patient records and a diagnostic examination. By sensing these fluid accumulation conditions, the invention enables a dynamic adaptation of sleep therapy during sleep.

In relation to the anatomical structure being affected by the fluid shift/accumulation, there are three major types of fluid shift, namely liquid accumulation in the lung, cardiac volume overloading and tissue swelling in the upper airway.

The system may be designed to detect and address any of these types of fluid shift. The system comprises a sensor arrangement for detecting parameters which are indicative of the type of fluid shift being detected. The sensor arrangement comprises one or multiple sensors which sense the type of fluid shift and the severity. The positional therapy implemented by the system may be one of various possible therapies which may be used in various combinations.

The system is able to balance the treatment of the sleep apnea itself with addressing issues arising from fluid accumulation, which may for example be side effects which result from those other sleep apnea therapies.

The sensor arrangement may comprise:
a bioimpedance sensor for mounting against the chest thereby to enable identification of fluid accumulation in the lungs.

Thus, a first type of fluid accumulation is fluid accumulation in the lungs. This may be detected at least in part using bioimpedance sensing.

The sensor arrangement may additionally comprise a blood oxygen saturation sensor for detecting a ventilation perfusion mismatch.

The sensor arrangement may further comprise a breathing sensor for monitoring a respiratory flow pattern.

The breathing sensor may be used to enable identification of atrial fluid accumulation.

Thus, a second type of fluid accumulation is fluid accumulation or volume overloading in the heart chambers and pulmonary/systemic veins. This may be detected at least in part using analysis of breathing flow patterns and the analysis of the pulsatility and flow patterns in a PPG signal.

The sleep therapy system may then further comprise an adaptive support ventilation system, and wherein the breathing sensor (i.e. respiratory flow sensor) is part of the adaptive support ventilation system, wherein the controller is adapted to trigger the detection of body position or the position of part of the body (such as the head or chinbone) based on the respiratory flow pattern. An adaptive support system may then be used to elevate the head and/or tilt the bed or mattress. A system may also be used to advance the mandible.

Thus, the system makes use of a respiratory flow sensor, for instance of a ventilation support system or a nasal or oral flow sensor or an abdominal and chest belt. Based on monitoring the sleeping posture or head position, the patient can then be triggered to change posture if an alternative posture is better, or a system may be used to change automatically the posture of the patient.

The sensor arrangement may comprise a hemodynamic sensor (ECG and/or PPG sensor) thereby to enable detection of cardiac stress.

Cardiac stress is caused by cardiac volume overloading (result from a change of venous return flow, increased vascular resistance, or a decline of ejection fraction and stroke volume), and thus consequently the change of the pulmonary intravascular pressure relates to fluid movement and fluid accumulation in the lung.

This cardiac stress may be manifested as a decline in stroke volume (SV) and ejection fraction (EF). These conditions may for example be determined based on measurements of heart rate, heart rate variability and blood pressure.

The controller may in this case be adapted to instruct a change in sleep position to a right side. This reduces cardiac volume overload. The heart is at the left side in the chest, so that in a right lateral body position the heart is at an elevated position. This change of the hydrostatic pressure impedes the venous return flow (filling of right atrium) and the stroke volume.

The sensor arrangement may further comprise a breathing sensor for monitoring a respiratory flow pattern to enable detection of breathlessness. The breathing sensor is for example adapted to detect Cheyne-Stokes respiration or hypoventilation.

This respiration can be detected based on respiratory movements such as chest movements and respiratory flow pattern. It is a typical compensatory effect in chronic heart failure patients.

The controller may be adapted to instruct a change in sleep position to the opposite side when Cheyne-Stokes respiration is detected. If the venous return flow declines, the system may for example trigger a positional change to the left lateral position. In case of an increased venous return flow the system may trigger a positional change to the right lateral position.

The sensor arrangement may comprise:
an ECG sensor for detecting volume overload;
a heart rate sensor;
an oxygen saturation sensor;
a bioimpedance sensor for identifying rostral fluid shift.

The heart rate sensor could be a PPG sensor, for measuring pulse rate and oxygen saturation SpO2.

The sleep therapy system may further comprise a breathing ventilation system, and wherein the controller is further adapted to control the breathing ventilation system in response to the identified fluid accumulation in a region of the body of the subject. Thus, the position therapy system is one part of a combination of therapies.

The fluid accumulation may indeed be an unintended side effect of the breathing ventilation therapy particularly in comorbid patients. The breathing ventilation system may be an AVAPS system or a CPAP system.

The controller is for example adapted to control the breathing ventilation system provide increased ventilation of the lung compartments, to improve/rebalance the mismatch in ventilation/circulation.

The invention also provides a method of controlling a sleep therapy system, comprising:
receiving detected physiological parameters of a subject;
identifying, from the physiological parameters, fluid accumulation in a region of the body of the subject;
receiving a detected sleeping position of the subject; and
control an alert system to instruct a change in sleep position of the subject thereby to remedy the fluid accumulation.

The invention also provides a computer program comprising computer program code which is adapted, when said program is run on a computer, to implement the method defined above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a first example of a sleep therapy system;
Figure 2 shows a second example of a sleep therapy system; and
Figure 3 shows a third example of a sleep therapy system.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a sleep therapy system, comprising a sensor arrangement for detecting physiological parameters of a subject, a detection system for detecting a sleeping position of the subject and an alert system for instructing a change in sleep position of the subject. From the monitored physiological parameters, fluid accumulation is identified in a region of the body of the subject. The alert system is controlled to instruct a change in sleep position thereby to address e.g. reverse the fluid accumulation. The position therapy may reduce the fluid accumulation or it may improve the patient condition in other ways. For example, pneumonia is a possible cause of edema. Pneumonia is caused by an infection which causes a local edema. Position therapy cannot get rid of the edema, but other compartments may be better ventilated and/or better perfused.

The invention is based on the recognition that for an optimized sleep apnea therapy, both the physiological stress during a sleep apnea event and the physiological stress caused by the therapy itself should both be considered in order to achieve the best therapy for the patient. The invention is based on the use of positional therapy to address these stress conditions. In particular, the invention involves sensing physiological stress by detecting the consequent fluid shift (e.g. volume accumulation). A therapy goal is then to avoid and mitigate the therapy-related stress condition. If a fluid shift is detected, the aim is for example to counteract and rebalance the fluid shift.

The physiological causes of fluid shifts and the implications for a sleep apnea therapy device will now be described in more detail.

As outlined above, there are various possible causes of fluid accumulation and fluid shifts, such as:
(i) Positive airway pressure provided by PAP (e.g. CPAP) or AVAPS therapy;
(ii) Increased negative inspiratory pressure caused by increased airway flow resistance (change in venous return flow);
(iii) Positive end expiratory pressure (PEEP) caused by hyperinflation (for example for COPD patients or during the third trimester in pregnancy);
(iv) Hydrostatic pressure changes, such as a fluid shift from the lower body compartments (legs) to upper body compartments (airway, neck) when going to bed, resulting from the positional change from upright to laying down.

Based on the location of a fluid shift or a volume accumulation and in relation to a comorbidity of the patient, a single therapy or a combination of different therapies may need to be provided.

As also mentioned above, the positional therapy which is the subject of this invention may be one of various possible therapies which may be used in various combinations. The position therapy may involve:
Control of body position or posture, for example to provide an elevated position of the head in order to lower a fluid shift from a lower body compartment; and/or
Control of body position or posture, for example to control a position of the thorax in order to improve ventilation of all compartments of the lung.

Additional therapies which may be used in combination with the positional therapy include:
Positive pressure therapy, in which the therapy pressure may be adapted in dependence on the detected fluid accumulation; and/or
Mandibular advancement, to reduce the airway flow resistance in the pharyngeal and oropharyngeal airway.

The system for example has respective subsystems to sense and monitor the fluid shift at a specific anatomical structures or in a compartment within one structure. The three anatomical structures of interest are:
The pulmonary airway;
The heart chambers and vascular structures in the thorax;
The upper airway.

As discussed in more detail below, different types of sensors may be used to detect these different types of fluid accumulation, i.e. fluid shift from one part of the body to another, either directly or by surrogate means. The analysis and post processing of the sensor data may be used to determine whether the fluid shift is above a threshold. The fluid shift may then be considered to be the result of physiological stress which needs to be addressed. The three types of fluid accumulation will now be discussed in turn.

### 1. Liquid accumulation in the lung during sleep in apnea patients

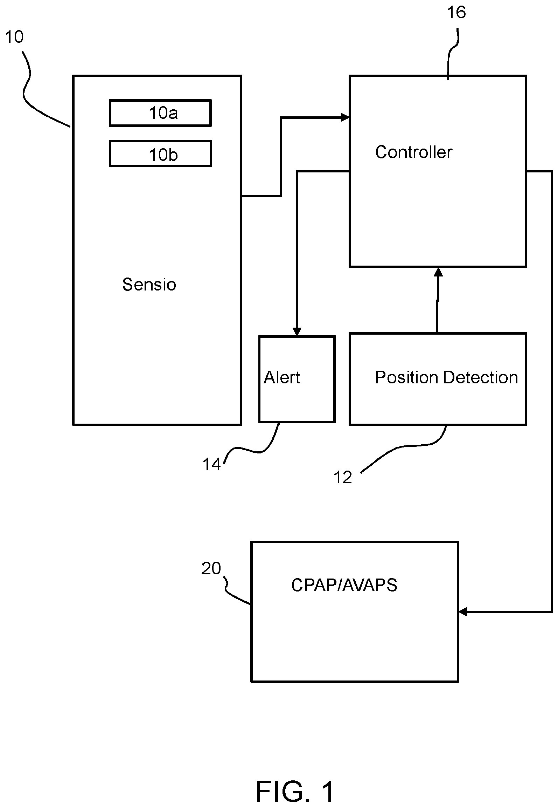

Figure 1 shows an example of a system which adapts a position therapy in order to detect and react to fluid accumulation in the lungs.

The system comprises a sensor arrangement 10 for detecting physiological parameters of a subject, a detection system 12 for detecting a sleeping position of the subject (e.g. the body and thorax position) and an alert system 14 for instructing a change in sleep position of the subject. The detection system is for example an accelerometer or optionally a camera. A controller 16 identifies, from the physiological parameters, fluid accumulation in the lungs and controls the alert system 14 to instruct a change in sleep position thereby to remedy the fluid accumulation.

The controller 16 optionally may also control the settings applied by one or more other therapy systems 20 such as CPAP system or an AVAPS system.

The alert system triggers a position change based on the location/compartment of the liquid accumulation (derived from the bio-impedance sensors) and the severity of liquid accumulation (derived from flow data). The positional change is performed in a way to elevate the compartment to a higher position to decrease the hydrostatic transmural pressure. For example, the change in position triggered by the system may be to a lateral position such that a fluid shift occurs into the inferior body side (after positioning) due to the change in hydrostatic pressure. When the uptake of fluid is detected in this side, a further positional change may then be triggered to the opposite lateral side.

In this example the sensor arrangement 10 comprises:
An array of bioimpedance sensors 10a integrated in belts or superficial patches worn at the chest. They sense a decrease in impedance that is specific for a liquid accumulation. The data from the array of sensors is used to localize the effected lung compartment and quantifies the liquid uptake. The bioimpedance sensors enable analysis of the fluid uptake by analyzing the relative change of the impedance. The blood oxygen saturation (SpO2) can be additionally measured by a PPG sensor to detect a ventilation perfusion mismatch. In OSA patients, the mismatch is predominantly caused by an insufficient ventilation and it is the primary target in the therapy. In heart failure patients, the insufficient circulation contributes further to the desaturation and thus has to be addressed by an advanced therapy approach.

An air flow sensor 10b measures changes in lung compliance which is specific for liquid/fluid accumulation. This may be a nasal/oral flow sensor to sense the inspiration and expiration flow pattern.

In the decision support to trigger a body position change, the data of the sensor is used as follows:
The array of bio-impedance sensors localize the compartment with increased fluid accumulation. The sensors can localize the fluid uptake but cannot quantify the accumulated liquid.

The airflow sensor measures the change in lung compliance. The change in lung compliance allows a quantified analysis of the volume of accumulated liquid.

These sensors are preferably complemented by an ECG and PPG sensor to measure the mismatch in ventilation and circulation.

If the above measured data are beyond predefined thresholds, a positional therapy is triggered.

In addition to providing positional therapy control, other therapy settings may be adapted. Thus, there is also the option to trigger an adapted breathing pattern, which ventilates compartments with a sufficient blood perfusion.

For example, the therapeutic pressure in a CPAP therapy may be reduced during expiration. A lowering of the expiratory pressure decreases the pneumatic stenting. It enables a better recoiling of the chest and it increases the pulmonary compliance and mitigates a further liquid accumulation. The pulmonary vascular resistance then decreases. As a cardiac response to this reduced vascular resistance, the pulmonary blood pressure and the heart rate will decrease. These can be measured by hemodynamic sensors as feedback indicative of therapy success. A ventilation pattern with increased chest breathing may be triggered to improve the ventilation of the upper compartments of the lung with a better blood perfusion.

Generally, nocturnal desaturation is the result of a ventilation-perfusion mismatch in the lung. This is caused either by an insufficient uptake of air (reduced minute air volume) or an insufficient pulmonary blood flow (reduced cardiac output). Even when the respiratory and hemodynamic parameters seem to be fine, the body can desaturate. This happens when only those compartments of the lung are ventilated which are not sufficiently perfused. This can happen when these compartments of the lung are obstructed by a pulmonary edema, namely a swelling of the tiny bronchi and alveoli. The bronchi and alveoli are flexible, highly compliant structures. A liquid accumulation will change the compliance, and hence the capacitive behavior of the lung. The liquid accumulation also has some, but less, impact on the resistive behavior.

Respiration in rest requests a muscular activity during inspiration. In rest, the expiration is a passive outflow of the air forced by the chest recoiling. The flow pattern is patient specific and depends on the body position. The patient specific flow pattern can be measured in advance in a sleep study. These measurements are a baseline for the detection of relative changes in the inspiratory and expiratory airflow.

In an upright position, a lung edema starts in the lower compartment of the lung. This compartment is then less perfused. When people lay down and fall asleep the breathing becomes more shallow. It becomes predominantly a diaphragm breathing which ventilates better the lower compartment of the lung, which on the other hand is also the compartment where the edema builds up during the day in upright position.

A relative change in the flow pattern is an indication for a change in the pulmonary airway, and in particular in the alveoli. The capacitive behavior can be measured by determining a decline of the residual volume and based on the dynamics of the recoiling chest. The first derivative of the expiratory flow and the frequency spectrum is specific for a change in the capacitive behavior.

To provide an effective therapy for a lung edema, the area with an increased fluid uptake should be identified, and a fluid reabsorption or a fluid shift into other compartments should be triggered by a position therapy system.

Thus, a first measure, which may be obtained by a processing unit is a measure of change in the capacitive behavior. If the change is above a predefined index, a change in position is triggered to mitigate a further accumulation of liquid in the lung. This results in a change in the hydrostatic conditions and can thus trigger a fluid shift.

This pulmonary accumulation also increases the pulmonary vascular resistance. The cardiac system compensates for this by increasing the pulmonary blood pressure and the heart rate. The changes in the pulmonary circulation and ventilation are both specific and complement each other in the therapy decision.

In summary, a liquid uptake in the pulmonary airway is caused primarily by an increased transmural pressure in the lung. There are two transmural pressures to be considered: the air pressure in the alveoli and the blood pressure in the pulmonary capillaries. The liquid accumulation contributes to a pulmonary edema.

### 2. Cardiac volume overloading

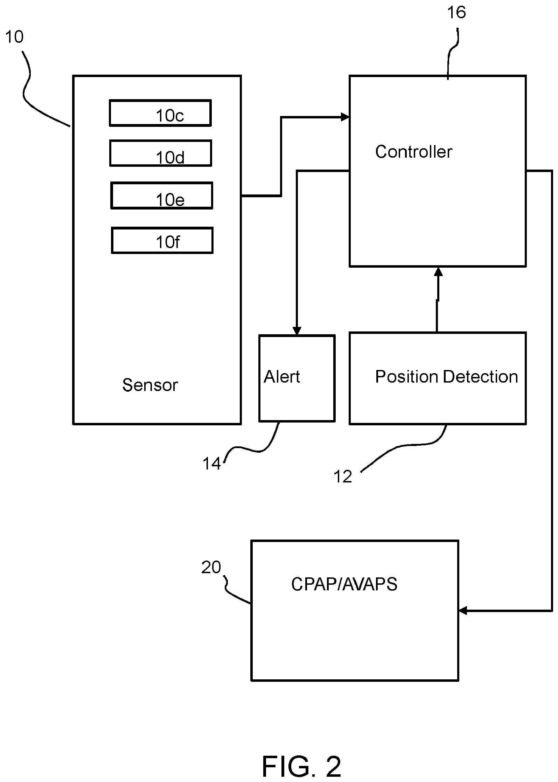

Figure 2 shows an example of a system which adapts a position therapy in order to detect change in venous return flow and atrial loading.

The system is the same as in Figure 1, but in this example the sensor arrangement 10 comprises:
A PPG sensor 10c to measure the shift in ejection fraction; the change in the pulsatility is specific for a change in stroke volume, and to measure the blood oxygen level;
An ECG sensor 10d to measure the change of heart rate, heart rate variability; which is specific for a change of the stroke volume and the modulation of the stroke volume by the respiration cycle;
Air flow sensor 10e;
A chest belt 10f to sense respiration drive.

In the decision support to trigger a body position change, the data of the sensor is used as follows:
The analysis of the PPG signal in the time domain provides specific data to quantify the change in the stroke volume.

The variability of the heart rate is specific for the modulation of ejection fraction and stroke volume. It is a surrogate means to analyze the baroreceptor activation by the atrial (over)loading.

The airflow sensor complements the measurement. The Cheyne-Stokes Respiration (CSR) flow pattern is specific for an atrial overloading.

If the above measured data are beyond predefined thresholds, a positional therapy is triggered.

The detection system 12 is in particular to sense the position of the thorax.

As described above, a change in the atrial loading is sensed by baroreceptors in the atrium. These Bainbridge sensors sense the stretching (dilating) of the atrium which corresponds to the loading/venous return flow. The sympathetic activity as a response to the Bainbridge receptors is twofold: an increase of the heart rate and the heart rate variability and an increase of respiration drive (change of tidal volume).

When the air flow sensor detects a CSR pattern, the ECG sensor detects a change in HR and HRV, the PPG sensor detect a variability in stroke volume and oxygen desaturation a processing unit will quantify the data. These quantified data are correlated with prerecorded data stored in a personalized data base. If the changes are above predefined threshold, a body position therapy will be triggered and the CPAP pressure settings are adapted accordantly.

The controller for example triggers a positional change from a left lateral position to a right lateral position to decrease the hydrostatic pressure. The hydrostatic pressure correlates with the filling pressure of the right atrium which is a surrogate measure for the venous return flow.

Typical in heart failure patients is a blood volume accumulation, resulting in a decline in stroke volume (SV) and ejection fraction (EF). A change of the intrathoracic pressure impedes the venous return flow, the ejection fraction and stroke volume. In a supine position and during sleep the health condition can worsen.

It can be caused by:
a change in the cardiac preload or afterload, or
an atrial overloading and dilated atrium, or
a decline in contractibility, or
a cardiac infarction or other coronary diseases.

The cardiac system tends to compensate by an increased heart rate and blood pressure. In sleep and in particular in sleep apnea patients, the pressure becomes strongly negative in the obstructed airway. This negative pressure impedes preload/afterload and the atrial filling. This has a strong impact on SV and EF. A change in SV and EF can be measured indirectly (surrogately) based on heart rate, heart rate variability and blood pressure and photoplethysmography (PPG).

If these hemodynamic parameter changes are above respective predefined thresholds the positional therapy can be adapted in a similar manner as explained above. In particular, the body position has an impact on the cardiac preload (atrial filling pressure is lower in the right lateral position). This changes the atrial fluid accumulation and the pulmonary circulation.

The positional therapy can be used to decrease the apnea-hypopnea index (AHI) and oxygen saturation index (OSI). Furthermore, by lowering the needed mean PAP pressure, it can improve the venous return flow and the pulmonary flow thereby decrease CSA events for example by lowering the Adaptive support ventilation AVAPS pressure settings.

Indeed, AVAPS is the preferred ventilation support therapy for CSA patients. A high peak pressure is uncomfortable and a major reason for low therapy compliance, and pressure related claustrophobia is typical in breathless persons. A high pressure is a hemodynamic burden, impeding the venous return, the atrial filling, increasing the pulmonary arterial pressure, contributing to lung edema, decreasing the stroke volume and cardiac output.

Thus, device settings in a CPAP device or an AVAPS device can also be modified to reduce the pressure related implications on preload/afterload. The therapy outcome will rebalance SV and EF, which are again surrogately measured using the heart rate and blood pressure and PPG.

An AVAPS device may be used to monitor the respiratory flow pattern, and if the pressure and the flow pattern are beyond predefined thresholds, the body position will be sensed. The system then creates an alarm if the respiratory pressure/flow patterns are above predefined values and the body is detected by the position therapy system to be in a supine and/or left lateral position. The position therapy system can then trigger movement to the right lateral side. As the human heart is on the left side in the chest, when a person is lying on the left side the hydrostatic filling pressure in the right atrium is a few mmHg higher compared to lying on the right side. Typically the Central Venous Pressure CVP is about 5 mmHg in healthy persons.

In patients with heart insufficiencies suffering from dilated atria this difference in the filling pressure has an impact on the return flow into the right atrium. This impedes the ejection fraction and the stroke volume.

The change in position may be triggered by the position therapy system (Philips Night Balance for instance) or it may use features of another therapy system such as a vibrating element embedded in the mask of the AVAPS device or a high frequency modulation of the pressure provided by the AVAPS device.

In an example, the positional therapy system may comprise hemodynamic sensors integrated into a chest belt. An ECG sensor may be used to detect volume overloading by heart rate changes, or a PPG sensor may be used to detect stroke volume changes. A PPG sensor can also detect desaturation during sleep. A bioimpedance sensor may also be used to measure a rostral fluid shift

In addition to detecting cardiac stress as explained above, a breathing sensor may be used to detect changes in breathing pattern which are specific for persons who feel breathless. For example, it is debated in the medical community that Cheyne - Stokes Respiration, CSR, is a compensatory effect in congestive heart failure patients (CHF). Respiratory movements may for example be measured by sensors integrated as part of the belt (e.g. using respiratory inductance plethysmography).
A surrogate measure may be obtained from am accelerometer used for body position detection.

Thus, in addition to detecting cardiac stress, a position change may be triggered based on detection of specific breathing patterns such as CSR. In this case, the position therapy may favor repositioning to left side.

### 3. Tissue swelling in the upper airway

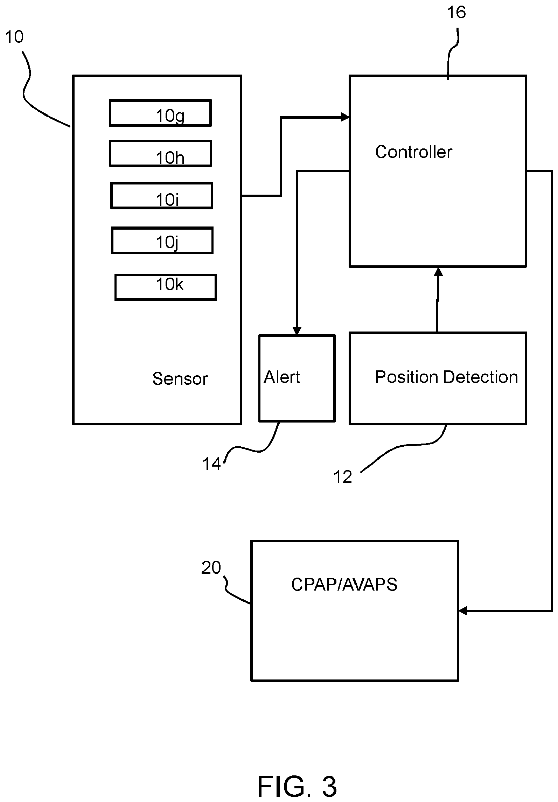

Figure 3 shows an example of a system which adapts a position therapy in order to detect and react to airway tissue welling.

The system is the same as in Figure 1, but in this example the sensor arrangement 10 comprises:
An air flow sensor 10g (a nasal/oral flow sensor) to sense airway occlusion and hypoventilation. The change of the expiratory flow pattern is specific for the increase of the flow resistance. The increased flow resistance correlates to the decrease of cross-section of the upper airway;
A chest belt 10h to sense respiration drive;
Bioimpedance sensors 10i at the nostril and the neck, and optionally also at the lower body extremities (legs). The quantified decrease in tissue impedance correlates to the narrowing of the airway. A database may be used to compare with bioimpedance values with recent values during a preceding time period;
A PPG sensor 10j and an ECG sensor 10k to sense heart rate and blood oxygenation (desaturation).

In the decision support to trigger a body position change, the data of the sensor is used as follows:
The analysis of the bio-impedance sensors are specific for the liquid accumulation and the narrowing of the airway.

The analysis of the flow sensor in the expiration cycle is specific for an increased flow resistance of the upper airway.

The chest and abdominal belts measure the respiration drive/work of breathing. The increased respiration drive is a surrogate for increased sympathetic activity. The increased sympathetic activity impedes the sleep quality.

The PPG complements the measurement. A desaturation is specific for this disorder resulting from a decreased airflow caused by an airway occlusion (short term alterations of flow pattern) or by tissue swelling (long term alteration of flow pattern).

If the above measured data are beyond predefined thresholds, a positional therapy is triggered.

The change in position triggered by the system is for example that the patient will be either alerted to reposition the body as defined in a protocol, or a motorized system will elevate the head, tilt the bed and change the bending of the neck.

In heart failure patients, intracellular liquid is accumulated in the lower comportment of the body when the person is in an upright position. When the patient lies down for sleep, the accumulated liquid from the lower body compartments will be redistributed due to a change in the position related hydrostatic pressure.

Thus, a liquid uptake in the upper airway is caused primarily by the rostral fluid shift from the lower body compartment to upper compartments. The liquid uptake in the nasal airway, tonsils and pharyngeal airway decreases the cross-section of the upper airway.

Some of the intracellular liquid can contribute to a swelling of the soft tissue in the airway (nose, palate, pharynx). This will narrow the upper airway and can be measured by an increased flow resistance during inspiration and expiration.

A flow pattern processing unit may be used to analyze these flow patterns to distinguish between an airway narrowing by an OSA event in inspiration and an airway narrowing by tissue swelling. The rostral fluid shift is a slow process that may take an hour. The narrowing of the airway in an OSA event will happen within one or several breath cycles.

A processing unit records the flow changes. Based on the timely behavior it distinguish between fluid shift and OSA related airway narrowing.

The change in position therapy for example involves instructing the patient to from supine to a lateral side in the case of an obstructive airway collapse. The instruction may be to elevate the head and chest in the case of tissue swelling in order to lower the hydrostatic pressure, which is a cause for the liquid accumulation in the airway.

As in the examples above, in addition to controlling the position therapy, other therapy systems may be controlled. In the case of a CPAP/BiPAP therapy the inspiratory CPAP pressure is increased to treat an obstructive event. The CPAP pressure will instead be decreased during expiration in case of a soft tissue swelling.

### 4. General overview

It is clear from the examples above that various sensors may be used to sense fluid shifts (either directly or by surrogate means), depending on the type of disorder and comorbidity of the patient, and hence the nature of the physiological stress they will experience as a result of the therapy being administered.

More generally, examples of different sensors that may be used in various combinations are:
Bioimpedance sensors, for example located at the neck, lower extremities, and/or chest;
An airflow pattern analyzer, for example for determining:
   the inspiration/expiration ratio;
   dynamics in inspiration and expiration;
   the airflow resistance in the upper airway (nasal and pharyngeal airway narrowing); and/or
   the airflow resistance in the lower airway, such as constriction of bronchi (i.e. asthma) and collapse of alveoli (COPD) and swelling of alveoli (pulmonary edema);
A chest belt and/or abdominal belt to measure a respiration pattern;
An ECG sensor and/or PPG sensor to sense heart rate, heart rate variability and SpO2;
A sensor for sensing stroke volume (SV) and cardiac output (CO) alterations;
A blood pressure sensor;
An EEG sensor;
An EMG sensor.

A sensor for measuring the venous pulsation, to sense changes in venous return at the jugular vein.

The venous return flow and its alterations can be measured surrogately. The increased venous return activates baroreceptors (Bainbridge receptors) which will increase the heart rate. The change of the volume (per heart cycle) - the stroke volume changes - can be surrogately measured by a PPG signal.

Various physiological conditions can be identified from combinations of these sensed parameters. For example, the sympathetic response to cardiac volume accumulation may for example be derived from analysis of heart rate, heart rate variability changes, blood pressure and pulse transit time. The baroreceptor response in cardiac volume overloading may be derived from analysis of the heart rate and blood pressure.

Various approaches are possible for inducing patient movement during sleep. Persistent feedback may be provided until the patient moves to the desired posture. Multiple feedback units (e.g. vibration units) may be placed on different places of worn item such as a belt and progressively triggered, to "guide" the patient to the desired posture, e.g. the right or left side.

The system is preferably able to detect each of the three types of fluid shift discussed in detail above. The fluid shift in the three anatomical structures (upper airway, pulmonary airway and cardiac chambers) are thus all diagnosed and quantified by the sensors and processing units described above. A decision support unit may then prioritize a positional change corresponding to the most severe fluid shift/liquid accumulation. Optionally the decision support unit may provide a timing protocol to trigger different positional changes in a sequential manner.

In use of the system, the patient cohort is for example already diagnosed in a sleep study and is a candidate for an OSA/CSA therapy. In a regular sleep study, the severity of OSA is known and quantified by the AHI value. In the PSG the effectiveness of the CPAP therapy is validated and CPAP is prescribed as the first line therapy.

However, in comorbid patients the implication of the CPAP therapy is not studied with respect to the comorbidity. In particular, the risk of a fluid accumulation and volume overloading and the exacerbation of the health condition caused by the fluid shift is not diagnosed. Furthermore, the impact of body position and the displacement of anatomical structures is not considered in the progression of fluid shift.

By way of example, particularly in OSA comorbid CHF patients, the progression in pulmonary edema and pneumonia worsens the health condition and may cause a hospital readmission. The overall target of the therapy is to stop or slow down the fluid accumulation in the lung. Positional therapy is one element in the therapy adaptation, and other therapies may also be adapted. The patient stratification will determine which is the primary therapy and which other therapies complement the primary one.

A sleep study in OSA patients with comorbidities becomes a multifactorial study. Primarily, the therapy goal is to rebalance the ventilation. Secondarily, it will study the side effect of the selected therapy with respect to fluid shift and its implication on the comorbidity. Based on the stratification in a sleep study, a positional therapy alone or in combination with other therapies (e.g. CPAP) may be proposed to optimize the treatment at home.

At home, if the sensors detect an ongoing or proceeding fluid shift, the settings of the therapy devices will be adapted, in particular, this invention relates to an approach by which a change of the body position will be triggered. The adaptions of the therapy settings are recorded and caregivers will be alerted in case the sensed fluid shift is beyond predefined thresholds.

The aim of the improvement of the sleep therapy provided by the invention is to keep the physiological burden as low as possible to avoid a disease exacerbation during treatment. In particular, this is particularly relevant for congestive heart failure (CHF) and COPD patients after release from hospital. In particular, it is important to avoid a hospital readmission in the first 30 days after release from the clinical ward.

More generally, the invention is of particular interest for patients with both pulmonary and cardiac diseases. The pulmonary diseases for example may include COPD, asthma and/or pulmonary hypertension. The cardiac diseases (and insufficiencies) may include right heart insufficiencies, left heart insufficiencies, systemic hypertension and/or cardiac arrhythmias.

After patient stratification, which identifies the type of comorbid health condition and the type and location of fluid shifts, an adapted therapy system is provided comprising at least positional therapy, but typical comprising a suitable combination of positional therapy, positive airway pressure therapy and repositioning of anatomical structures.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A sleep therapy system, comprising:
a sensor arrangement (10) for detecting physiological parameters of a subject;
a detection system (12) for detecting a sleeping position of the subject;
an alert system (14) for instructing a change in sleep position of the subject; and
a controller (16),
wherein the controller is adapted to:
identify, from the physiological parameters, fluid accumulation in a region of the body of the subject; and
control the alert system to instruct a change in sleep position thereby to remedy the fluid accumulation.

2. The system of claim 1, wherein the sensor arrangement comprises:
a bioimpedance sensor for mounting against the chest thereby to enable identification of fluid accumulation in the lungs.

3. The system of claim 2, wherein the sensor arrangement comprises a blood oxygen saturation sensor for detecting a ventilation perfusion mismatch.

4. The system of any one of claims 1 to 2, wherein the sensor arrangement comprises a breathing sensor for monitoring a respiratory flow pattern.

5. The system of claim 4, wherein the sleep therapy system further comprises an adaptive support ventilation system, and wherein the breathing sensor is part of the adaptive support ventilation system, wherein the controller is adapted to trigger the detection of body position based on the respiratory flow pattern.

6. The system of any one of claims 1 to 5, wherein the sensor arrangement comprises:
a hemodynamic sensor thereby to enable detection of cardiac stress.

7. The system of claim 6, wherein the controller is adapted to instruct a change in sleep position to a right side.

8. The system of claim 6, wherein the sensor arrangement further comprises a breathing sensor for monitoring a respiratory flow pattern to enable detection of breathlessness.

9. The system of claim 8, wherein the breathing sensor is adapted to detect Cheyne-Stokes respiration.

10. The system of claim 9, wherein the controller is adapted to instruct a change in sleep position to a left side when Cheyne-Stokes respiration is detected.

11. The system of any one of claims 6 to 10, wherein the sensor arrangement comprises:
an ECG sensor for detecting volume overload;
a heart rate sensor;
an oxygen saturation sensor; and
a bioimpedance sensor for identifying rostral fluid shift.

12. The system of any one of claims 1 to 11, wherein the sleep therapy system further comprises a breathing ventilation system, and wherein the controller is further adapted to control the breathing ventilation system in response to identified fluid accumulation in a region of the body of the subject.

13. The system of claim 12, wherein the controller is adapted to control the breathing ventilation system provide increased chest breathing.

14. A method of controlling a sleep therapy system, comprising:
receiving detected physiological parameters of a subject;
identifying, from the physiological parameters, fluid accumulation in a region of the body of the subject;
receiving a detected sleeping position of the subject; and
control an alert system to instruct a change in sleep position of the subject thereby to remedy the fluid accumulation.

15. A computer program comprising computer program code which is adapted, when said program is run on a computer, to implement the method of claim 14.
